# EUROPEAN PATENT SPECIFICATION

(11) **EP 3 326 581 B1**
(45) Date of publication and mention of the grant of the patent: **19.01.2022**
(21) Application number: 16827451.2
(22) Date of filing: 03.02.2016
(51) Int. Cl.: A61F 2/07

(54) **STENT GRAFT**
STENTIMPLANTAT
ENDOPROTHÈSE COUVERTE

(30) Priority: 23.07.2015 JP 2015145831
(43) Date of publication of application: 30.05.2018
(73) Proprietor: Japan Lifeline Co., Ltd., Tokyo 140-0002 (JP)
(72) Inventor: SAKAI, Masamune, Tokyo 140-0002 (JP); HURUYA, Hideki, Tokyo 140-0002 (JP)
(74) Representative: Piotrowicz, Pawel Jan Andrzej
(86) International application number: PCT/JP2016/053172
(87) International publication number: WO 2017/013885

(56) References cited:
- EP-A2- 1 666 005
- WO-A1-01/15633
- WO-A1-96/28115
- JP-A- 2008 513 118
- JP-A- 2015 501 173
- US-A1- 2005 240 261
- US-A1- 2008 208 325
- US-A1- 2009 088 828

## Description

### Technical Field

The invention relates to a stent graft that is used upon treatment, for example, of arterial dissection (an aneurysm), etc.

### Background Art

In recent years, in a case where a blood vessel, or any other tubular organ in vivo is narrowed or occluded, a technique (stent placement) has been used that makes it possible to expand or retain a lumen by placing a stent at an affected area. The stent includes a wire member (a wire), etc. In particular, a stent graft has been used upon treatment, for example, of arterial dissection, etc., in a blood vessel. The stent graft is provided with a tubular graft that covers the stent described above. (For example, reference is made to Patent Literature 1.)

Further, for example, Patent Literature 2 discloses, as a stent graft for a thoracic aorta, a stent graft that is to be inserted from a base of a leg (a groin) of a patient and delivered to an affected area (a diseased part).

### Citation list

### Patent Literature

Patent Literature 1: Japanese Unexamined Patent Application Publication No. 2008-99995
Patent Literature 2: Japanese Unexamined Patent Application Publication (Translation of PCT Application) No. 2007-503923
Patent Literature 3: WO 96/28115 A1 describes a radially expandable stent-graft and method of making the same, including at least one stent member encapsulated between at least two longitudinally expanded polytetrafluoroethylene (ePTFE) coverings. Patent literature 4: EP 1 666 005 discloses a stent graft comprising a graft layer and a membrane layer substantially surrounding the graft layer whereby said graft layer has a first average permeability and the membrane layer has a second average permeability less than the first average permeability and is adapted to restrict flow of fluids through the wall.

### Summary of Invention

When a stent graft is to be inserted from a groin in a manner described above, it is necessary to mount a stent graft having an outer diameter, for example, from about 20 mm to about 46 mm on a fine catheter having a diameter, for example, from about 20 Fr (frenches) to about 26 Fr. Accordingly, it is necessary to decrease the outer diameter of the stent graft in a state after the diameter of the stent graft is reduced. Therefore, a graft of the stent graft typically has a small thickness (is typically thin).

Now, an open stent graft (OSG) method has been proposed recently as one of methods for treating, for example, arterial dissection in a thoracic aorta, etc. In the OSG method, the aorta is incised after a thoracic part is opened, and the stent graft is inserted from the incised part. Further, base end side of the stent graft is sutured to a vessel of a patient to make an anastomosis therebetween. Further, an anastomosis may be made, on an as-needed basis, between the foregoing anastomosis part and another artificial blood vessel other than the foregoing stent graft.

In a case of performing a procedure by utilizing the foregoing OSG method, the graft may be insufficient in strength when the graft has a small thickness as described above. This may possibly cause a rip in the graft upon the suture, which makes it difficult to make the anastomosis, for example. Further, for example, this may cause an increase in leakage of blood from the graft (in particular, leakage of blood from a pinhole made upon making the anastomosis between the graft and the blood vessel of the patient or the artificial blood vessel). This may possibly lead to insufficiency in the treatment of the arterial dissection, etc. It is therefore desired to make a proposition that makes it possible to improve convenience in treatment such as that described above.

The invention has been made in view of such a concern, and it is an object of the invention to provide a stent graft that is able to improve convenience in treatment.

A stent graft of the invention includes: a tubular graft; and at least one stent that is disposed in a region corresponding to part or all of the graft, and includes one or a plurality of wire members. The graft has a thickness that is greater than a diameter of at least one of the one or the plurality of wire members of the at least one stent. In the stent graft of the invention, the graft has liquid retainability that is greater on outer circumferential surface side of the graft than on inner circumferential surface side of the graft. In this case, it is easier to make an anastomosis between the graft and a blood vessel of a patient or an artificial blood vessel. In addition, attachment characteristics between the graft and an inner circumferential surface of the blood vessel are improved. Moreover, it is difficult for a blood clot to be generated on the inner circumferential surface side of the graft.

In the stent graft of the invention, the graft has the thickness that is greater than the diameter of at least one of the one or the plurality of wire members of the at least one stent. As a result, it is possible to decrease the possibility of ripping of the graft, or to suppress leakage of blood from the graft, for example, upon treatment of arterial dissection, etc. utilizing the OSG method.

In the stent graft of the invention, in a case where the graft is disposed to cover at least outer circumference side of the at least one stent, a part, of at least one of the one or the plurality of wire members, corresponding to half or more of the diameter of the relevant wire member may be embedded in inner circumferential surface side of the graft. In this case, it may be easier for a circumference of the stent to be surrounded by the graft, for example, upon expansion of the stent graft. As a result, it becomes difficult for the inner circumference side of the stent to be protruded from an inner circumferential surface of the graft, which makes it difficult for a blood flow inside the stent graft to be disrupted. Moreover, it is easier for a force applied by the stent to be dispersed at the graft (an effect of absorbing the force by the graft is increased), which prevents inside of a blood vessel from being damaged by the outer circumference side of the stent.

In the stent graft of the invention, the at least one stent may be disposed in a region corresponding to part of the graft and extending in an axis direction of the graft. In this case, it is easier to make the anastomosis between the stent graft and the blood vessel of the patient or the artificial blood vessel by utilizing a region, of the graft, in which the stent is not disposed.

According to the stent graft of the invention, the graft has the thickness that is greater than the diameter of at least one of the one or the plurality of wire members of the at least one stent. It is therefore possible to decrease the possibility of ripping of the graft, or to suppress the leakage of blood from the graft, for example, upon the treatment of arterial dissection, etc. utilizing the OSG method. Hence, it is possible to improve convenience in treatment.

According to the stent graft of the invention, the stent graft may be composed of woven material.

### Brief Description of Drawings

[FIG. 1] FIG. 1 is a schematic perspective view of an outline configuration example of a stent graft according to one embodiment of the invention.
[FIG. 2] FIG. 2 is a schematic cross-sectional view of a detailed configuration example of the stent graft illustrated in FIG. 1.
[FIG. 3] FIG. 3 is a schematic diagram illustrating an example of a method of using the stent graft illustrated in FIG. 1.
[FIG. 4] FIG. 4 is a schematic diagram illustrating an example of a state of placement of the stent graft illustrated in FIG. 1.
[FIG. 5] FIG. 5 is a schematic cross-sectional view of a configuration example of a stent graft according to a comparative example.
[FIG. 6] FIG. 6 is a schematic cross-sectional view of a configuration example of a stent graft according to Modification example 1.
[FIG. 7] FIG. 7 is a schematic perspective view of an outline configuration example of a stent graft according to Modification example 2.

### Description of Embodiments

Some embodiments of the invention are described in detail below, in the following order, with reference to drawings.

### 1. Embodiment (an example in which a stent is disposed in a region corresponding to part of a graft)

### 2. Modification Examples

Modification Example 1 (an example in which the graft covers both inner circumference side and outer circumference side of the stent)
Modification Example 2 (an example in which the stent is disposed in a region corresponding to all of the graft)

### 3. Other Modification Examples

### [1. Embodiment]

### [Outline Configuration]

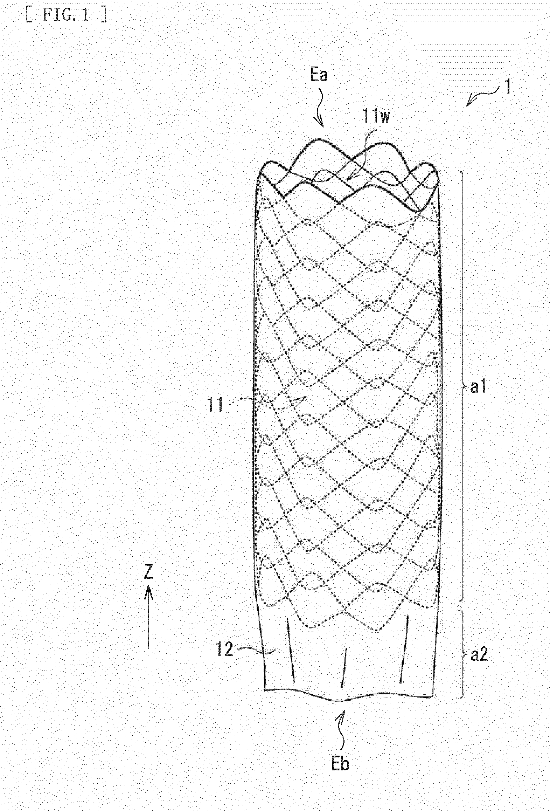

FIG. 1 is a schematic perspective view of an outline configuration example of a stent graft (a stent graft 1) according to one embodiment of the invention. The stent graft 1 may be an instrument that is used, for example, upon treatment of arterial dissection, etc. by utilizing the OSG method. The stent graft 1 is to be placed at a part to be treated (for example, inside a blood vessel such as an aorta), as will be described later.

Referring to FIG. 1, the stent graft 1 has a tubular (cylindrical) structure that extends along its own axis direction (a Z-axis direction). The stent graft 1 includes a stent 11 and a graft 12. It is to be noted that the stent graft 1 has a length in the axis direction, for example, from about 2 cm to about 30 cm. Further, the stent graft 1 has an outer diameter at the time of its expansion, for example, from about 6 mm to about 46 mm.

### [Stent 11]

Referring to FIG. 1, the stent 11 includes one or a plurality of wire members (wires) 11w, and has a tubular (cylindrical) structure in this example. Specifically, in the example illustrated in FIG. 1, the tubular structure is configured by a mesh structure. Further, the tubular mesh structure is provided by weaving the one or the plurality of wire members 11w into a predetermined pattern. It is to be noted that examples of the patterns of weaving may include plane weave, twill weave, or stockinet. Alternatively, the tubular mesh structure may be provided by disposing one or more wire members 11w that are bent in a zigzag form and processed into a tubular shape.

Moreover, the stent 11 is disposed in a region corresponding to part of the graft 12 and extending in the axis direction of the graft 12, in this example. In other words, the stent graft 1 has a stent disposed region a1 and a stent non-disposed region a2 in its own axis direction. The stent disposed region a1 is a region in which the stent 11 is disposed. The stent non-disposed region a2 is a region in which the stent 11 is not disposed. It is to be noted that, in this example, the stent non-disposed region a2 is provided on side of an end Eb of the stent graft 1, and the stent disposed region a1 extends to an end Ea of the stent graft 1 on the end Ea side of the stent graft 1, as illustrated in FIG. 1. The end Eb is one end of the stent graft 1, and the end Ea is the other end of the stent graft 1. It is to be noted that a length of the stent 11 in its own axis direction (a length of the stent disposed region a1) is, for example, from about 2 cm to about 25 cm.

In this example, a metal wire member may be preferable as a material of the wire member 11w described above. In particular, a shape-memory alloy may be preferably employed. The shape-memory alloy is provided with a shape-memory effect, superelasticity, etc. by a thermal process. However, stainless-steel, tantalum (Ta), titanium (Ti), platinum (Pt), gold (Au), tungsten (W), etc. may be used as the material of the wire member 11w, depending on the application. As the foregoing shape-memory alloy, for example, an alloy of nickel (Ni) and Ti, an alloy of copper (Cu), zinc (Zn), and X (X is aluminum (Al), iron (Fe), etc.), an alloy of Ni, Ti, and X (X is Fe, Cu, vanadium (V), cobalt (Co), etc.), etc. may be preferably used. It is to be noted that, for example, synthetic resin, etc. may be used as the foregoing wire member 11w. Moreover, a metal wire member having a surface covered with Au, Pt, etc. by a method such as plating may be used as the wire member 11w. Alternatively, a complex wire member in which a core material is covered with an alloy may be used as the wire member 11w. The core material includes a material that does not transmit radiation, such as Au or Pt.

### [Graft 12]

The graft 12 has a tubular (cylindrical) shape as illustrated in FIG. 1. The graft 12 is so disposed as to cover (coat) part or all of the stent 11. Specifically, the graft 12 is so disposed as to cover outer circumference side of the stent 11 (the wire member 11w) in this example.

Moreover, the graft 12 is coupled to the stent 11 by a method such as sewing, attaching, or welding. In this case, the graft 12 covers the stent 11 and is coupled to the stent 11 so as not to influence expansion and contraction of the stent 11. It is to be noted that a coupling part at which the graft 12 and the stent 11 are coupled to each other is provided appropriately at a location such as at both ends of the stent 11 or in the middle of the stent 11.

Examples of a material that may be used as the graft 12 described above include: thermoplastic resin that is formed in to a tubular shape by a molding method such as extrusion molding or blow molding; a tubular-shaped knitted or woven material including a fiber of thermoplastic resin or a super-fine metal wire; a tubular-shaped non-woven fabric including thermoplastic resin or super-fine metal; a tubular-shaped flexible resin sheet or a tubular-shaped porous sheet; a structure derived from resin that has been dissolved in a solvent and is formed into a thin tubular shape by electrospinning; etc.

In this example, as the foregoing knitted or woven material, a publicly-known material knitted or woven in plane weave, twill weave, etc. may be used. Alternatively, a knitted or woven material with a crimp such as that subjected to a crimping process may also be used. It is to be noted that, in particular, the tubular-shaped knitted or woven material of a fiber of thermoplastic resin may be preferable, and the tubular-shaped material woven in twill weave of the fiber of the thermoplastic resin may be more preferable among the foregoing materials. One reason for this is that such a material is superior in strength, pore rate, productivity, etc.

Moreover, as the thermoplastic resin described above, for example, resin having durability and causing a small tissue reaction may be used. Examples of the resin having durability and causing a small tissue reaction include: polyolefin such as polyethylene, polypropylene, or an ethylene-α-olefin copolymer; polyester such as polyamide, polyurethane, polyethylene terephthalate, polybutylene terephthalate, polycyclohexane terephthalate, or polyethylene-2, 6-naphthalate; fluororesin such as polyethylene fluoride or polypropylene fluoride, etc. It is to be noted that, among the foregoing materials, the polyester such as polyethylene terephthalate or the fluororesin such as polyethylene fluoride or polypropylene fluoride that are chemically stable, have great durability, and cause a small tissue reaction may be preferably used in particular.

### [Detailed Configuration]

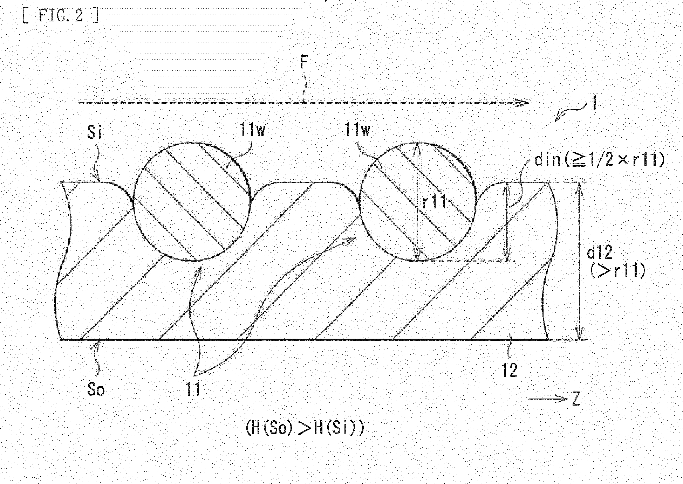

Next, referring to FIG. 2, a description is given on a detailed configuration example of the stent graft 1 illustrated in FIG. 1. FIG. 2 is a schematic cross-sectional view of the detailed configuration example of the stent graft 1.

In the stent graft 1, firstly, the graft 12 has a thickness d12 that is greater than a diameter r11 of the wire member 11w of the stent 11 (d12 > r11), as illustrated in FIG. 2. In other words, the graft 12 has a structure with a greater thickness (a thick structure) compared to an existing typical graft (for example, a graft 102 according to a comparative example described later). It is to be noted that the foregoing thickness d12 of the graft 12 is from about 0.15 mm to about 0.60 mm (for example, 0.4 mm). The foregoing diameter r11 of the wire member 11w is from about 0.10 mm to about 0.50 mm (for example, 0.3 mm).

Moreover, as illustrated in FIG. 2, in the stent 11, a part, of the wire member 11w, corresponding to half (1/2) or more of the diameter r11 of the wire member 11w is embedded in side of an inner circumferential surface Si of the graft 12. In other words, din ≥ {(1/2) × r11} is satisfied as illustrated in FIG. 2 where din represents an embedded length, i.e., a length (a depth) of the part, of the wire member 11w, that is embedded in the inner circumferential surface Si side of the graft 12. It is to be noted that, to give an example, in the foregoing example case where the diameter r11 of the wire member 11w is 0.3 mm, a part corresponding to two-thirds of the wire member 11w (the embedded length din equals about 0.2 mm) is embedded in the inner circumferential surface Si side of the graft 12.

Moreover, the graft 12 has liquid retainability H(So), on side of an outer circumferential surface So of the graft 12, that is greater than liquid retainability H(Si), on the inner circumferential surface Si side of the graft 12 (H(So) > H(Si)), as illustrated in FIG. 2. In other words, liquid permeability on the outer circumferential surface So side of the graft 12 is greater than liquid permeability on the inner circumferential surface Si side of the graft 12. In a case where the graft 12 has porous characteristics (the graft 12 includes the foregoing porous sheet, etc.), each of the liquid retainability and the liquid permeability of the graft 12 described above is defined by magnitude of a voidage on the outer circumferential surface So side and the inner circumferential surface Si side of the graft 12. In other words, the higer the voidage is, the greater each of the liquid retainability and the liquid permeability becomes. In contrast, the lower the voidage is, the smaller each of the liquid retainability and the liquid permeability becomes.

### [Workings and Effects]

The stent graft 1 is able to expand or retain a lumen by being placed at a part to be treated (for example, inside a blood vessel such as an aorta) upon treatment, for example, of arterial dissection, etc. of a patient. Further, in particular, the stent graft 1 is used, for example, upon treatment utilizing the OSG method that is one of the methods of treating arterial dissection at a thoracic aorta, etc.

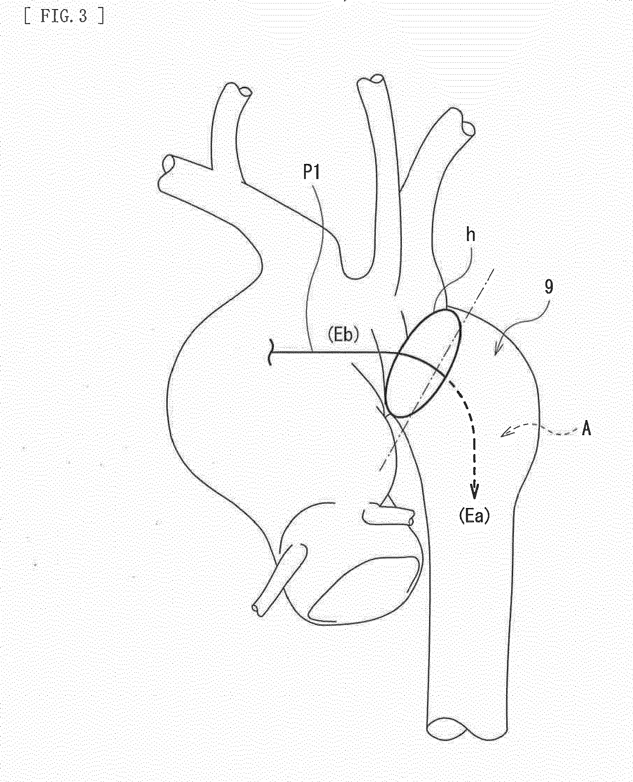

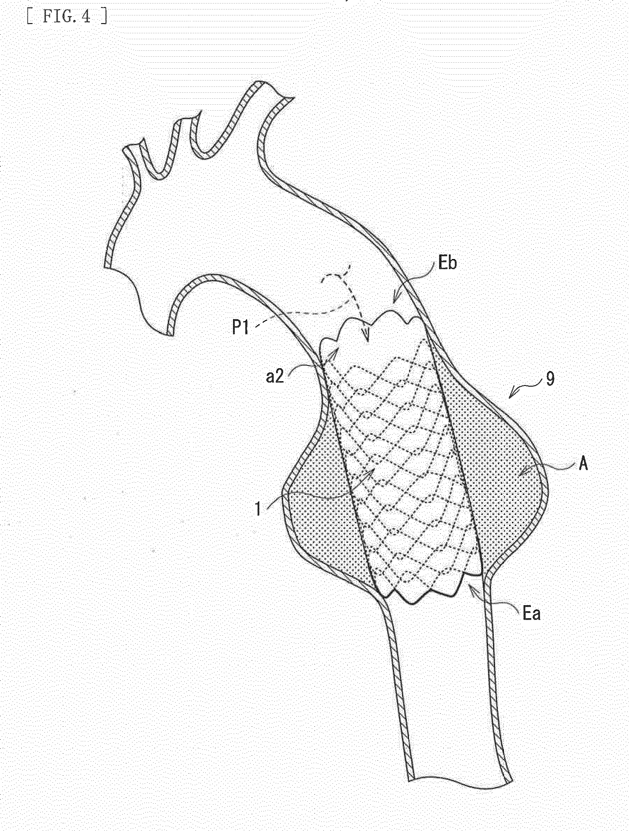

Referring to FIGs. 3 and 4, a description is given next of an overview of the method of treating arterial dissection (an aneurysm), etc. by utilizing the OSG method. FIG. 3 is a schematic diagram illustrating an example of a method of using the stent graft 1 upon the foregoing treatment. FIG. 4 is a schematic diagram illustrating an example of a state of placement of the stent graft 1 upon the foregoing treatment. It is to be noted the following description refers to an example case where an artery 9 (a thoracic aorta) that is a blood vessel to be treated is a descending aorta. Further, FIGs. 3 and 4 each illustrate an aneurysm at the artery 9 to be treated as an aneurysm A.

Referring to FIG. 3, in the OSG method, first, the stent graft 1 reduced in diameter is inserted from an opening "h" with the use of an unillustrated instrument dedicated to a predetermined delivery, after a thoracic part of a patient is opened (see an arrow PI). The opening h derives from incision of part of the artery 9. On this occasion, the stent graft 1 is so inserted that the end Ea of the stent graft 1 is on tip end side, and the end Eb (the stent non-disposed region a2 side) is on base end side, as illustrated in FIG. 3. Thereafter, the end Ea of the stent graft 1 is caused to reach a part located farther than a part to be treated in the artery 9 (a part located farther than a part near a location provided with the aneurysm A), by the use of the delivery-dedicated instrument.

Further, the diameter of the stent graft 1 is increased by utilizing self-expanding force of the stent graft 1. As a result, the stent graft 1 is fixed to an inner wall of the artery 9, as illustrated in FIG. 4. This expands or retains the lumen of the artery 9 near the location provided with the aneurysm A. Thereafter, an anastomosis is made between the base end side (the end Eb side) of the stent graft 1 and the artery 9 (the blood vessel of the patient) by suturing. Further, another anastomosis may be made between the foregoing anastomosis part and another artificial blood vessel different from the stent graft 1, on an as-needed basis.

An inner circumference of the aneurysm A is covered by the stent graft 1 as illustrated in FIG. 4 in the foregoing manner. This allows blood to flow through inside of the stent graft 1, and prevents the aneurysm A from being influenced by a blood pressure, etc. Hence, it is possible to prevent an increase in diameter of the aneurysm A and a rupture of the blood vessel at the aneurysm A. It is also possible to maintain a blood flow at the aneurysm A.

Moreover, the foregoing treatment method utilizing the OSG method has the following advantages in particular, compared with a treatment method (an existing treatment method) that inserts a catheter from a base of a leg (a groin) of a patient and thereby delivers a stent graft to a part to be treated. That is, it is possible to perform a procedure on a part having an important branch (for example, an aortic arch) which has caused extreme difficulty in procedure by the existing treatment method. Moreover, when compared with a method that replaces a removed diseased part by an artificial blood vessel, and makes an anastomosis on each side of the artificial blood vessel, fixing by the stent graft 1 is performed instead of suturing of the descending aorta (instead of making an anastomosis on terminal side). In other words, the OSG method omits making the anastomosis between the tip end side (the end Ea side) of the stent graft 1 and the descending aorta. It is therefore easier to make an anastomosis. This reduces time for operation (extracorporeal circulation time), and avoids opening of a left thoracic part or a great thoracic incision that is necessary for the suturing of the descending aorta. Therefore, operative invasion on the patient is reduced (a burden on the patient is reduced upon the treatment). Moreover, the OSG method makes it possible to set a transplantation range of the artificial blood vessel to a great range. This makes it possible to perform a surgical procedure for a complication near the treated part, which is also advantageous. In addition, the stent graft applied to the OSG method is not introduced from a groin, unlike the foregoing existing treatment method. This eliminates the necessity to cause the stent graft to pass through a fine blood vessel. It is therefore possible for the stent graft to have an outer diameter that is great (thick) to some extent even in a state where the stent graft is reduced in diameter.

### [Comparative Example]

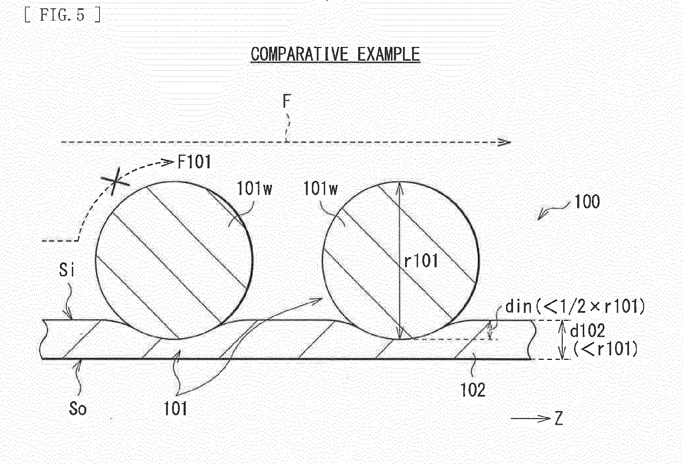

FIG. 5 is a schematic cross-sectional view of a configuration example of a stent graft (a stent graft 100) according to a comparative example. The stent graft 100 according to the comparative example corresponds to the stent graft 1 according to the embodiment illustrated in FIGs. 1 and 2 except that a magnitude relationship in size between the stent 11 and the graft 12 is changed (a stent 101 and a graft 102 are provided in place of the stent 11 and the graft 12). Other configuration of the stent graft 100 is basically similar to that of the stent graft 1.

Specifically, in the stent graft 100, the graft 102 has a thickness d102 that is smaller than a diameter r101 of a wire member 101w of the stent 101 (d102 < r101), as illustrated in FIG. 5. In other words, the graft 102 has a structure that has a thickness as small as possible. One reason for this is that the stent graft 100 is applied to the existing treatment method described above.

Specifically, in a case where the stent graft 100 is inserted from a groin of a patient, it is necessary to mount the stent graft 100 on an unillustrated fine catheter having a diameter, for example, from about 20 Fr to about 26 Fr. Accordingly, it is necessary to make small the outer diameter of the stent graft 100 in a state after the diameter of the stent graft 100 is reduced. The thickness d102 of the graft 102 of the stent graft 100 is therefore set to be as small as possible (as thin as possible). It is to be noted that the thickness d102 of the graft 102 described above is from about 0.05 mm to about 0.15 mm (for example, 0.10 mm), and the diameter r101 of the wire member 101w is from about 0.20 mm to about 0.50 mm (for example, 0.45 mm).

Therefore, the following concerns may arise in a case where the stent graft 100 according to the comparative example is applied to, for example, the OSG method. Specifically, in a case where arterial dissection (an aneurysm), etc. is treated by utilizing the OSG method, for example, the graft 102 may be possibly ripped upon suturing of the end of the stent graft 100 or at any other timing, which causes difficulty in making an anastomosis. One reason for this is insufficiency in strength of the graft 102 in a case where the thickness d102 of the graft 102 is small (d102 < r101) as described above. Further, for example, leakage of blood from the graft 102 may be increased, which may possibly cause insufficiency in treating the arterial dissection, etc. In particular, the small thickness d102 of the graft 102 causes an increase in leakage of blood from a pinhole caused upon making an anastomosis between the graft 102 and a blood vessel of the patient or an artificial blood vessel. This may cause insufficiency in blocking blood flowing into a false lumen of the aneurysm or the arterial dissection, which may possibly cause insufficiency in an effect of the treatment. Therefore, the stent graft 100 according to the comparative example may decrease convenience, for example, upon the treatment of the arterial dissection, etc. utilizing the OSG method.

### [Embodiment]

In contrast, in the stent graft 1 according to the embodiment, the thickness d12 of the graft 12 is greater than the diameter r11 of the wire member 11w of the stent 11 (d12 > r11), as illustrated in FIG. 2. In other words, compared with the graft 102 according to the comparative example illustrated in FIG. 5, the graft 12 has a structure with a greater thickness. This makes it possible for the stent graft 1 to reduce the possibility of ripping of the graft 12 upon the suturing of the end Eb, etc., upon the treatment of the arterial dissection, etc. utilizing the foregoing OSG method, for example. Further, this makes it also possible for the stent graft 1 to suppress the leakage of blood from the graft 12 or the pinhole described above, upon the treatment of the arterial dissection, etc. utilizing the foregoing OSG method, for example.

Moreover, in the stent graft 1, the graft 12 is so disposed as to cover at least the outer circumference side of the stent 11 as illustrated in FIG. 2. Further, the part, of the wire member 11w of the stent 11, corresponding to half or more of the diameter r11 of the wire member 11w is embedded in the inner circumferential surface Si side of the graft 12, as illustrated in FIG. 2. In other words, the embedded length din ≥ {(1/2) × r11} is satisfied, as illustrated in FIG. 2.

Accordingly, for example, when the stent graft 1 expands, it is easier for a circumference of the stent 11 to be surrounded by the graft 12. This makes it difficult for the inner circumference side of the stent 11 to be protruded from the inner circumferential surface Si of the graft 12. It is therefore difficult for the blood flow inside the stent graft 1 to be disrupted (for example, see a blood flow F illustrated in FIG. 2). In contrast, it is to be noted that the stent graft 100 according to the comparative example described above has a state where the embedded length din < {(1/2) × r11} is established, as illustrated in FIG. 5. Therefore, it is easy for the inner circumference side of the stent 101 to be protruded from the inner circumferential surface Si of the graft 102, which makes it easier for a blood flow inside the stent graft 100 to be disrupted (for example, see a blood flow F101 and a × (cross) mark illustrated in FIG. 5).

Moreover, a force applied from the stent 11 is easily dispersed at the graft 12 having the great thickness (an effect of absorbing the force by the graft 12 is increased). This prevents inside of the blood vessel from being damaged by the outer circumference side of the stent 11. In particular, in the case of arterial dissection, a wall of a blood vessel at a diseased part is extremely weak. It is therefore extremely easy for the wall of the blood vessel to be ripped. Hence, what is important is that it is possible to prevent the inside of the blood vessel from being damaged as in the case of the stent graft 1.

Moreover, in the stent graft 1, the liquid retainability H(So) on the outer circumferential surface So side of the graft 12 is greater than the liquid retainability H(Si) on the inner circumferential surface Si side of the graft 12 (H(So) > H(Si)), as illustrated in FIG. 2. In other words, the liquid permeability on the outer circumferential surface So side of the graft 12 is greater than the liquid permeability on the inner circumferential surface Si side of the graft 12.

Typically, blood flows smoothly on a surface of a graft having low liquid retainability. Therefore, the graft having low liquid retainability has an advantage that a blood clot is less likely to be generated. The graft having low liquid retainability includes, for example, a knitted or woven material that is knitted or woven tightly, or a film having low liquid transmission rate. However, such a graft has drawbacks that it is difficult to make an anastomosis between the graft and a blood vessel of a patient or an artificial blood vessel, and that such a graft has poor compatibility with a surrounding tissue (such a graft has low characteristics in attachment to the surrounding tissue).

In contrast, a graft having high liquid retainability has advantages that it is easy to make an anastomosis between the graft and a blood vessel of a patient or an artificial blood vessel, and that such a graft has high compatibility with a surrounding tissue (such a graft has high characteristics in attachment to the surrounding tissue). The graft having high liquid retainability includes, for example, a knitted or woven material that is knitted or woven roughly, or a sponge-like film. However, such a graft causes a lot of leakage of blood from the graft, and causes insufficiency in blocking of blood flowing into a false lumen of the aneurysm or the arterial dissection, which may possibly cause insufficiency in an effect of the treatment.

In contrast, in the stent graft 1 according to the embodiment, the outer circumferential surface So side of the graft 12 has high liquid retainability, and the inner circumferential surface Si side of the graft 12 has low liquid retainability, as described above. Therefore, the stent graft 1 according to the embodiment is able to have the advantages of each of the foregoing two types of structures. In other words, the stent graft 1 makes it easier to make an anastomosis between the graft 12 and a blood vessel of a patient or an artificial blood vessel, and increases attachment characteristics between the graft 12 and an inner circumferential surface of a blood vessel (the graft 12 has higher compatibility with a surrounding tissue). Further, the stent graft 1 makes it difficult for a blood clot to be generated on the inner circumferential surface Si side of the graft 12.

According to the embodiment, the liquid retainability and the liquid permeability described above is achievable owing to the structure of the graft 12 that has the thickness greater than that of the existing graft (the thickness d12 of the graft 12 is greater than the diameter r11 of the wire member 11w of the stent 11), as described above. In other words, it is the structure of the graft 12 having the great thickness that allows for the above-described difference in structure between the outer circumferential surface So side of the graft 12 and the inner circumferential surface Si side of the graft 12 (for example, the difference in voidage of the porous structure of the graft 12), to thereby achieve the bilayer structure. It is to be noted that, in contrast, the stent graft 100 according to the foregoing comparative example is so configured as not to show such liquid retainability and such liquid permeability (is so configured that the liquid retainability and the liquid permeability of the graft 102 are suppressed to be low), in order to reduce the thickness of the graft 102 as much as possible.

Moreover, in the stent graft 1 according to the embodiment, the stent 11 is disposed in the region (the stent disposed region a1) corresponding to part of the graft 12 and extending in the axis direction of the graft 12, as illustrated in FIG. 1. It is therefore easier to make an anastomosis between the end (the end Eb, in this example) of the stent graft 1 and the blood vessel of the patient or the artificial blood vessel by utilizing the stent non-disposed region a2 of the graft 12.

According to the present embodiment, the graft 12 has the thickness d12 that is greater than the diameter r11 of the wire member 11w of the stent 11, as described above. Therefore, the followings are achieved, for example. That is, it is possible to reduce a possibility of ripping of the graft 12, for example, upon treatment of arterial dissection, etc. utilizing the OSG method. Further, it is possible to reduce leakage of blood from the graft 12 or the pinhole described above, for example, upon the treatment of arterial dissection, etc. utilizing the OSG method. This makes it possible to sufficiently treat the arterial dissection, etc. Hence, it is possible to improve convenience in treatment.

### [2. Modification Examples]

A description is given next of some modification examples (Modifications examples 1 and 2) of the foregoing embodiment. It is to be noted that components that are the same as those in the embodiment are denoted with the same numerals, and will not be described further where appropriate.

### [Modification Example 1]

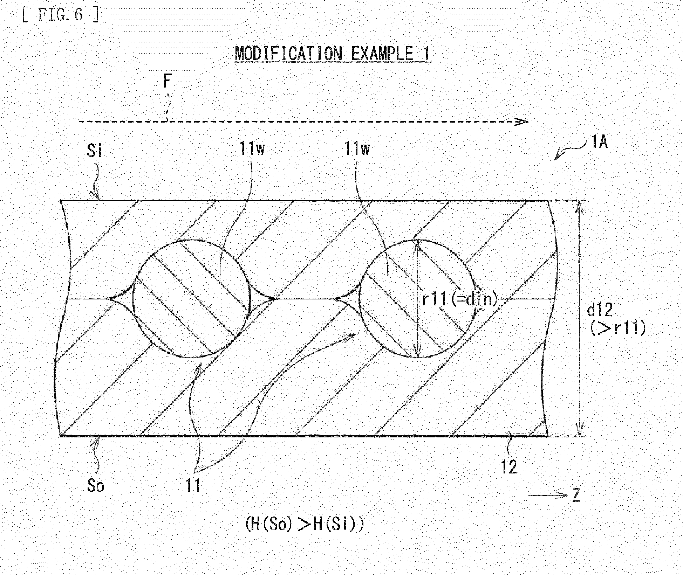

FIG. 6 is a schematic cross-sectional view of a configuration example of a stent graft (a stent graft 1A) according to Modification example 1. The stent graft 1A corresponds to the stent graft 1 illustrated in FIGs. 1 and 2 except that how the stent 11 is covered with the graft 12 is changed. Other configuration of the stent graft 1A is basically similar to that of the stent graft 1.

Specifically, in the stent graft 1 according to the embodiment, the graft 12 is so disposed as to cover the outer circumference side of the stent 11 (the wire member 11w), as illustrated in FIG. 2. In contrast, in the stent graft 1A according to the present modification example, the graft 12 is so disposed as to cover both the outer circumference side and the inner circumference side of the stent 11 (the wire member 11w), as illustrated in FIG. 6. Accordingly, in the present modification example, the embedded length din of the wire member 11w equals the diameter r11 of the wire member 11w (din = r11), as illustrated in FIG. 6.

It is possible, also according to the present modification example having the foregoing configuration, to achieve effects similar to those according to the foregoing embodiment by workings similar to those according to the foregoing embodiment basically.

### [Modification Example 2]

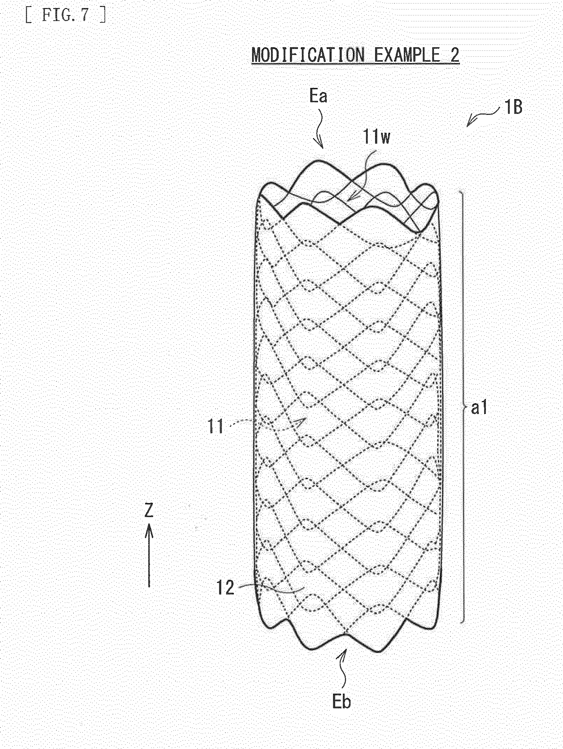

FIG. 7 is a schematic perspective view of an outline configuration example of a stent graft (a stent graft 1B) according to Modification example 2. The stent graft 1B corresponds to the stent graft 1 illustrated in FIGs. 1 and 2 except that a region in which the stent 11 is disposed is changed. Other configuration of the stent graft 1B is basically similar to that of the stent graft 1.

Specifically, in the stent graft 1 according to the embodiment, the stent 11 is disposed in the region corresponding to part of the graft 12 and extending in the axis direction of the graft 12, as illustrated in FIG. 1. In other words, the stent graft 1 has the stent disposed region a1 and the stent non-disposed region a2 in the axis direction of the stent graft 1. In contrast, in the stent graft 1B according to the present modification example, the stent 11 is disposed in a region corresponding to all of the graft 12 and extending in the axis direction of the graft 12, as illustrated in FIG. 7. In other words, the stent graft 1B includes only the stent disposed region a1 in the axis direction of the stent graft 1B, and does not include the stent non-disposed region a2. In other words, the stent disposed region a1 extends from one end Eb of the stent graft 1B to the other end Eb of the stent graft 1B.

It is possible, also according to the present modification example having the foregoing configuration, to achieve effects similar to those according to the foregoing embodiment by workings similar to those according to the foregoing embodiment basically.

### [3. Other Modification Examples]

The invention has been described above with reference to the embodiment and the modification examples thereof; however, the invention is not limited to the embodiment, etc. described above, and is modifiable in a variety of ways.

For example, shapes, locations, sizes, numbers, materials, etc., of the respective members described in the foregoing embodiment are non-limiting, and may respectively be any other shape, location, size, number, material, etc. Specifically, for example, the graft 12 may cover only the inner circumference side of the stent 11. Moreover, the arranged shape (the weaving pattern) of the wire member 11w of the stent 11 is not limited to those referred to in the foregoing embodiment, and may be any other arranged shape. Moreover, in some cases, the embedded length din of the wire member 11w of the stent 11 may be smaller than the half of the diameter r11 of the wire member 11w. In other words, din < {(1/2) × r11} may be satisfied. In addition, in a case where the plurality of wire members 11w are used for the stent 11, for example, the diameters r11 of the respective wire members 11w may be different from each other. In this case, it is sufficient that the diameter r11 of at least one of the plurality of wire members 11w satisfies each of the foregoing relational expressions. Moreover, although the foregoing embodiment, etc. are described with reference to an example case where only one stent 11 is disposed in the stent graft, this is non-limiting. Two or more stents 11 may be individually disposed in the stent graft (for example, the two or more stents 11 may be disposed in the axis direction while being separated from each other).

Moreover, although the foregoing embodiment, etc. have been described with reference to an example case where the liquid retainability (and the liquid permeability) on the outer circumferential surface So side of the graft 12 is greater than the liquid retainability (and the liquid permeability) on the inner circumferential surface Si side of the graft 12, this is non-limiting. That is, in contrast, the liquid retainability (and the liquid permeability) on the outer circumferential surface So side of the graft 12 is equal to or smaller than the liquid retainability (and the liquid permeability) on the inner circumferential surface Si side of the graft 12, in some cases.

Moreover, although the foregoing embodiment, etc. have been mainly described with reference to an example of the stent graft that is applied to the treatment of the descending aorta, this is non-limiting. That is, the stent graft of the invention is also applicable to treatment of a blood vessel such as any artery other than the descending aorta (for example, an ascending aorta, an aortic arch, a thoracoabdominal aorta, an abdominal aorta, an iliac artery, a femoral artery, etc.)

## Claims

1. A stent graft (1) comprising:
a tubular graft (12); and
at least one stent (11) that is disposed in a region corresponding to part or all of the graft (12), and includes one or a plurality of wire members (11w),
the graft (12) having a thickness (d12) that is greater than a diameter (r11) of at least one of the one or the plurality of wire members (11w) of the at least one stent (11),
wherein the graft (12) has liquid retainability that is greater on outer circumferential surface (So) side of the graft (12) than on inner circumferential surface (Si) side of the graft (12).

2. The stent graft (1) according to claim 1, wherein
the graft (12) is disposed to cover at least outer circumference side of the at least one stent (11), and
a part, of at least one of the one or the plurality of wire members (11w), corresponding to half or more of the diameter (r11) of the relevant wire member (11w) is embedded in inner circumferential surface (Si) side of the graft (12).

3. The stent graft (1) according to any one of claims 1 to 2, wherein the at least one stent (11) is disposed in a region (a1) corresponding to part of the graft (12) and extending in an axis direction (Z) of the graft (12).

4. The stent graft (1) according to any one of claims 1 to 3, wherein the graft (12) is composed of woven material.

## Patentansprüche

1. Stentimplantat (1), umfassend:
ein rohrförmiges Transplantat (12); und
mindestens einen Stent (11), der in einem Bereich angeordnet ist, der einem Teil oder der Gesamtheit des Implantats (12) entspricht, und ein oder eine Vielzahl von Drahtelementen (11w) einschließt,
wobei das Implantat (12) eine Dicke (d12) aufweist, die größer ist als ein Durchmesser (r11) von mindestens einem des einen oder der Vielzahl von Drahtelementen (11w) des mindestens einen Stents (11),
wobei das Implantat (12) eine Flüssigkeitsrückhaltefähigkeit aufweist, die auf der Seite der Außenumfangsoberfläche (So) des Implantats (12) größer ist als auf der Seite der Innenumfangsoberfläche (Si) des Implantats (12).

2. Stentimplantat (1) nach Anspruch 1, wobei
das Implantat (12) angeordnet ist, um mindestens die Seite des Außenumfangs des mindestens einen Stents (11) zu bedecken, und
ein Teil, von mindestens einem des einen oder der Vielzahl von Drahtelementen (11w), der der Hälfte oder mehr des Durchmessers (r11) des relevanten Drahtelements (11w) entspricht, in der Seite der Innenumfangsoberfläche (Si) des Implantats (12) eingebettet ist.

3. Stentimplantat (1) nach einem der Ansprüche 1 bis 2, wobei der mindestens eine Stent (11) in einem Bereich (a1) angeordnet ist, der einem Teil des Implantats (12) entspricht und sich in einer Achsenrichtung (Z) des Implantats (12) erstreckt.

4. Stentimplantat (1) nach einem der Ansprüche 1 bis 3, wobei das Implantat (12) aus gewebtem Material zusammengesetzt ist.

## Revendications

1. Endoprothèse couverte (1) comprenant :
une greffe tubulaire (12); et
au moins une endoprothèse (11) qui est disposée dans une région correspondant à une partie ou à la totalité de la greffe (12), et inclut un ou une pluralité d'éléments de fil (11w),
la greffe (12) ayant une épaisseur (d12) qui est supérieure à un diamètre (r11) d'au moins un parmi le ou la pluralité d'éléments de fil (11w) de l'au moins une endoprothèse (11),
dans laquelle la greffe (12) a une capacité de rétention de liquide qui est plus grande sur le côté de la surface circonférentielle externe (So) de la greffe (12) que sur le côté de la surface circonférentielle interne (Si) de la greffe (12).

2. Endoprothèse couverte (1) selon la revendication 1, dans laquelle
la greffe (12) est disposée pour couvrir au moins le côté circonférentiel externe de l'au moins une endoprothèse (11), et
une partie, d'au moins un parmi le ou la pluralité d'éléments de fil (11w), correspondant à la moitié ou plus du diamètre (r11) de l'élément de fil pertinent (11w) est noyée dans le côté de la surface circonférentielle interne (Si) de la greffe (12).

3. Endoprothèse couverte (1) selon l'une quelconque des revendications 1 à 2, dans laquelle l'au moins une endoprothèse (11) est disposée dans une région (a1) correspondant à une partie de la greffe (12) et s'étendant dans une direction axiale (Z) de la greffe (12).

4. Endoprothèse couverte (1) selon l'une quelconque des revendications 1 à 3, dans laquelle la greffe (12) est composée de matériau tissé.
